(19) **Europäisches Patentamt**

**European Patent Office**

**Office européen des brevets**

(11) **EP 0 857 973 B1**

(12) **EUROPÄISCHE PATENTSCHRIFT**

(45) Veröffentlichungstag und Bekanntmachung des Hinweises auf die Patenterteilung:
**20.10.2004 Patentblatt 2004/43**

(51) Int Cl.⁷: **G01N 33/68**, G01N 33/58, G01N 33/543

(21) Anmeldenummer: **98890007.2**

(22) Anmeldetag: **12.01.1998**

(54) **Verfahren zur Bestimmung eines Proteins mit einer Mutation**

Method for the determination of a protein with a mutation

Méthode pour la détermination d'une protéine avec une mutation

(84) Benannte Vertragsstaaten:
**AT BE CH DE DK ES FI FR GB IT LI NL SE**

(30) Priorität: **13.01.1997  AT  4497**

(43) Veröffentlichungstag der Anmeldung:
**12.08.1998   Patentblatt 1998/33**

(73) Patentinhaber: **Baxter Aktiengesellschaft**
**1221 Wien (AT)**

(72) Erfinder:
 • **Moritz, Berta, Dr.**
   **1180 Wien (AT)**
 • **Kiessig, Stephan**
   **69168 Wiesloch (DE)**
 • **Lang, Hartmut, Dr.**
   **2560 Grillenberg (AT)**
 • **Schenk, Verena**
   **69254 Malsch (DE)**

(74) Vertreter: **Alge, Daniel, Mag. Dr. rer.nat. et al**
**Sonn & Partner Patentanwälte**
**Riemergasse 14**
**1010 Wien (AT)**

(56) Entgegenhaltungen:
WO-A-92/00311      WO-A-94/09155
WO-A-95/21938      WO-A-96/02670
WO-A-96/03656      WO-A-96/19500

 • **KRUL E.S. ET AL.: "ApoB-75, a truncation of apolipoprotein B associated with familial hypobetalipoproteinimia: genetic and kinetic studies" J. LIPID RESEARCH, Bd. 33, 1992, Seiten 1037-1050, XP000892924**
 • **BERTINA, R.M. ET AL.: "Mutation in blood coagulation factor V associated with resistance to activated protein C" NATURE, Bd. 369, Nr. 6475, 5. Mai 1994 (1994-05-05), Seiten 64-67, XP000563812**
 • **TAKAMIYA O. ET AL.: "Dysfunctional factor VII variant (FVII Tondabayashi) with R79Q: determination of mutated site with monoclonal anti-human factor VII antibody (B101/B1)" CLINICAL CHEMISTRY, Bd. 44, Nr. 9, 1998, Seiten 1993-1995, XP002134877**

**Beschreibung**

Die vorliegende Erfindung betrifft ein Verfahren zur Bestimmung eines Proteins mit einer Mutation in einer Probe, die gegebenenfalls den Wildtyp des Proteins enthält, durch Zusetzen eines Liganden zur Probe, welcher Ligand entweder an das Protein mit der Mutation bindet oder an den Wildtyp, Ermitteln des Ausmaßes an gebundenem Liganden und Qualifizieren bzw. Quantifizieren der Mutation.

Die Diagnose von Mutationen bei Menschen konnte in der Vergangenheit oft nur über die entsprechenden pathologischen Manifestationen vorgenommen werden. Beispielsweise konnten durch Mutationen bedingte Stoffwechselstörungen erst viel zu spät durch die Folgen der nicht ausreichend metabolisierten Zwischenprodukte erkannt werden (z.B. Phenylketonurie oder Chorea Huntington).

Später wurden spezifische biochemische Tests, beispielsweise Enzymtests, für eine Vielzahl von verbreiteten Mutationen entwickelt, mit welchen eine frühere Erkennung von verschiedenen Erbkrankheiten, wie z.B. Chorea Huntington, Phenylketonurie, Alkaptonurie, etc. ermöglicht wurden. In diesen Enzymtests wurde entweder das nicht-metabolisierte Zwischenprodukt chemisch detektiert oder der jeweilige Stoffwechselprozess, bei welchem sich die Mutation manifestiert, in vitro nachgestellt. Diese Tests sind jedoch überaus aufwendig und kompliziert und darüberhinaus waren sie für jede Krankheit zum Teil höchst verschieden. Eine einfache Reihenuntersuchung, bei welcher verschiedene Krankheiten in gleichen oder ähnlichen Systemen diagnostiziert werden können, ist daher nur bei wenigen Erkrankungen möglich.

Die Einführung der PCR-Methodologie brachte eine große Rationalisierung auf dem Gebiet der Diagnose genetisch bedingter pathologischer Zustände. Die Diagnose von Mutationen war nunmehr nicht mehr von einer histologischen oder pathologischen Manifestation bzw. vom Vorliegen eines geeigneten biochemischen Testsystems abhängig, sondern konnte direkt am Erbmaterial des Probanden erkannt werden. Mit dieser Methodologie konnte nunmehr auch eine Reihe verschiedenster Mutationen nebeneinander diagnostiziert werden, wobei der Versuchsansatz - abgesehen von jeweils spezifischen Primerpaaren - im Wesentlichen gleich bleiben konnte.

Die PCR-Methodologie bedarf jedoch einer aufwendigen und ausgereiften Laboratoriums-Infrastruktur und -Logistik. So ist die Diagnose mittels PCR-Methodologie vom apparativen Aufwand her sehr kostenintensiv; man benötigt spezielle Thermocycler und Elektrophorese- bzw. Sequenzierapparaturen zur Detektion der PCR-Produkte. Daher ist die PCR-Detektionsmethodologie nicht in allen Diagnoselaboratorien installierbar. Vor allem in kleinen Laboratorien oder in Ländern, in welchen diese Technologie schlichtweg zu teuer ist, bedarf es anderer Diagnosesysteme bzw. wird noch an der alten biochemischen Diagnosemethode festgehalten.

Bei der Routinediagnostik erfordert PCR daher einen insgesamt derart hohen Aufwand, um eine hinreichende Zuverlässigkeit zu gewährleisten, dass sie nur in hochmodernen, genauen und gesicherten Laboratorien von speziell geschultem Personal durchgeführt werden kann. Dies ist jedoch in der Regel für einfache Diagnoselabors nicht möglich.

Die PCR-Technik alleine ist für den Nachweis von Mutationen wenig geeignet. Sie dient in diesem Falle eher der Probenvorbereitung, wobei das interessierende Gen amplifiziert wird. Anschließend erfolgt dann der Nachweis der Mutation durch Sequenzierung, beispielsweise durch Sequenzierung nach der Methode von Sänger.

Die WO 95/21938A betrifft ein Verfahren zur Detektion des Vorhandenseins eines genetischen Defekts, der mit Thrombose und/oder einer schwachen Anti-Koagulanz-Reaktion in Verbindung steht. Gemäß diesem Verfahren wird ein spezifischer Antikörper eingesetzt, der entweder an das mutierte Protein oder an das Wildtyp-Protein bindet. Das Verfahren kann dazu verwendet werden zu analysieren, ob eine Person homozygot oder heterozygot für eine Mutation ist, wobei die zu untersuchende Probe mit einer Probe einer normalen, nicht erkrankten Person, verglichen wird.

Die WO 92/00311A betrifft Proben zur Detektion eines mutanten p53-Proteins sowie ein Verfahren zur Unterscheidung zwischen dem Wildtyp p53- und mutanten p53-Protein sowie zwischen unterschiedlich mutierten p53-Proteinen. Hierbei werden beispielsweise markierte Antikörper verwendet, die an immobilisiertes mutantes bzw. Wildtyp-p53-Protein binden, (1-Schritt-Verfahren) bzw. nicht markierte Antikörper, die an das mutante bzw. Wildtyp-p53-Protein binden, wonach markierte Antikörper an die nicht markierten Antikörper binden (2-Schritt-Verfahren). Die Menge an gebundenem, markiertem Antikörper wird in einem weiteren Schritt gemessen. Es wurden weiters Proteine identifiziert, die nur an spezifische Mutanten binden. Demnach ist hier ein Verfahren beschrieben, wobei ein Ligand entweder an das Protein mit der Mutation oder an den Wildtyp bindet, um das Ausmaß an Mutant/Wildtyp zu ermitteln.

Die WO 94/09155A betrifft ein Verfahren zur Diagnose und Prognostizierung der Alzheimer-Erkrankung in einer Person, wobei der Nachweis einer Apolipoprotein E Typ 4 (ApoE4)Isoform durchgeführt wird. Eine Möglichkeit dieses Nachweises ist ein Antikörper-Assay mit einem Antikörper, der selektiv an ApoE4 bindet oder mit Antikörpern, die selektiv an ApoE2 und ApoE3 binden.

In Krul et al., J. Lipid Research, Bd. 33 (1992), Seiten 1037-1050, wird eine Studie beschrieben, wobei eine Mutation von ApoB in Subjekten mit Hypobetalipoproteinämie identifiziert wurde. Hierbei wurde ein Verfahren zur Detektion von spezifischen Isoformen mit Hilfe von monoklonalen Antikörpern beschrieben.

Die Aufgabe der vorliegenden Erfindung ist nun, eine neue Anwendung eines Verfahrens zur Diagnose von Mutationen in einer biologischen Probe zur Verfügung zu stellen, welches Verfahren einfach, standardisiert und wenig fehleranfällig sein soll und welches sich vor allem für Reihenuntersuchungen eignet.

Weiters soll mit diesem Verfahren auch eine Quantifizierung des Anteils an einem spezifischen mutierten Protein durchgeführt werden können.

Diese Aufgabe wird durch ein Verfahren der eingangs erwähnten Art gelöst, bei welchem das Protein der Blutgerinnungsfaktor VII ist.

Das eingangs erwähnte Verfahren eignet sich besonders gut zur Qualifizierung und Quantifizierung von Faktor VII-Mutanten. Hierbei eignet sich insbesondere die Mutation Arg353→Gln353 (Silveira et al., Thromb. Haemostas. 72 (5) (1994), 734 bis 739) für die Anwendung des obigen Verfahrens, welche mit einem erhöhten Risiko für kardiovaskuläre Erkrankungen assoziiert ist. Als möglicher Ligand kann beispielsweise eine Struktur verwendet werden, die zwischen dem Ersatz einer basischen Aminosäure durch eine neutrale Aminosäure unterscheidet.

Beim erfindungsgemäßen Verfahren wird nicht - wie bei der PCR-Methodologie - die Mutation im Genom, sondern in "klassischer" Weise anhand des Phänotyps, welcher in der Regel ein Protein ist, bestimmt.

Unter Wildtyp ist im Allgemeinen jene Form des Proteins zu verstehen, die normalerweise in einer entsprechenden Population vorliegt und die die derzeit diesem Protein zugeordnete Funktion zur Folge hat.

Unter einem Protein mit einer Mutation wird gemäß der vorliegenden Erfindung ein solches Protein verstanden, welches mindestens eine Änderung der Aminosäuresequenz gegenüber dem Wildtyp aufweist. Eine solche Änderung der Aminosäuresequenz kann beispielsweise in einem Austausch einer Aminosäure gegen eine andere Aminosäure (Punktmutation) bestehen oder durch andere bekannte Mutationsmechanismen, wie z.B. ein Frameshift, eine Änderung im Stoppcodon, eine Insertion oder Deletion, entstanden sein.

Gemäß der vorliegenden Erfindung werden bevorzugterweise Punktmutationen in einem Protein bestimmt aber auch solche, die funktionelle Mutationen darstellen, die also das jeweilige Protein in einer spezifischen Funktion beeinflussen. Die veränderte Funktion des Proteins wird gemäß der vorliegenden Erfindung auf die veränderten Bindungseigenschaften für Liganden zurückgeführt. In einer weiteren bevorzugten Ausführungsform werden kleinere Insertionen bzw. Deletionen mit einem Umfang von bis zu 100 Aminosäuren, mehr bevorzugt bis zu 30, am meisten bevorzugt bis zu 10 Aminosäuren, bestimmt. Im gegenständlichen Verfahren wird durch den Einsatz eines sehr spezifischen Liganden das Vorhandensein einer Mutation detektiert. Dieser Ligand bindet im erfindungsgemäßen Verfahren spezifisch entweder an die durch die Mutation betroffene Stelle im Protein oder er bindet spezifisch nur den Wildtyp.

Dadurch ist eine sehr spezifische Analyse einer Probe umfassend Faktor VII, ob eine bestimmte Mutation gegeben ist oder nicht, möglich, andererseits wird aber auch eine einfache Quantifizierung des Ausmaßes der Mutation ermöglicht, welche unter Zugrundelegung einer einfachen Bindungskinetik ermittelt werden kann. Aus diesem Grund ist es auch vorteilhaft, dass der Ligand eine möglichst hohe Affinität bzw. Avidität zur betroffenen Stelle im Protein aufweist, sodass eine schnelle und von langwierigen Gleichgewichtseinstellungen nicht betroffene Analyse gewährleistet werden kann. Als Ligand wird daher bevorzugterweise ein Peptid oder ein Proteinfragment zugegeben. Durch bekannte Methoden der Peptidkombinatorik können derart spezifische Peptide oder Proteinfragmente routinemäßig und sehr spezifisch für das jeweilige Protein bzw. die jeweilige Proteinstelle hergestellt werden. Die Auswahl eines geeigneten Liganden erfolgt in der Praxis beispielsweise derart, dass aus dem vom Wildtyp-Protein physiologischerweise gebundenem Liganden oder Reaktionspartner der Bindungsbereich ausgewählt wird, der von dem Protein mit Mutation nicht mehr erkannt wird. Diese geeignete Bindungsstelle kann beispielsweise mittels eines Peptid-Scans, wie noch in den Beispielen näher erläutert, ermittelt werden. Gleichwohl können aber auch Liganden zum Einsatz kommen, die organisch chemische Substanzen darstellen, welche die physiologische Raumstruktur eines entsprechenden Liganden nachbilden können. Solche Substanzen können beispielsweise Zuckerverbindungen, Nukleotide oder organische Ringverbindungen sein.

Eine bevorzugte Möglichkeit zur "Konstruktion" eines solches Liganden besteht darin, bestimmte Peptide oder modifizierte Peptide von einem physiologischen Bindungsprotein zum Wildtyp-Protein abzuleiten. Das Protein kann beispielsweise ein Enzym sein und der Ligand gemäß der vorliegenden Erfindung kann dann von einem entsprechenden Substrat abgeleitet sein. Als ein weiteres Beispiel für ein Protein-Liganden-Paar soll Faktor - Kofaktor angeführt werden. Allgemein handelt es sich bei dem Liganden um eine von einem Bindungspartner des Wildtyp-Proteins abgeleitete Struktur (Mimogand). Voraussetzung ist selbstverständlich, dass die Bindungsstelle des physiologischen Bindungspartners von der jeweiligen Mutation betroffen ist.

Wenn der Ligand ein Peptid ist, so umfasst es wenigstens drei Aminosäuren, wobei gemäß einer bevorzugten Ausführungsform wenigstens eine Aminosäure unphysiologisch ist. Beispielsweise umfasst der Ligand maximal 200 Aminosäuren, bevorzugt 3 bis 100 Aminosäuren, am meisten bevorzugt 5 bis 20 Aminosäuren.

Der Ligand kann auch ein Proteinfragment sein, beispielsweise ein Fab-Fragment, ein sogenannter «single-chain antibody" oder ein "mini-body". In einer bevorzugten Ausführungsform ist der Ligand kein Antikörper, der an die spezifische Mutation bindet.

Der erfindungsgemäße Test ermöglicht eine zuverlässige, schnelle und - weil es im Prinzip ein einfacher Bindungstest ist - wenig fehleranfällige Unterscheidung bzw. Bestimmung von Proben mit Wildtyp-Protein und Proben mit mutierten Proteinen.

Es ist dadurch auch leicht möglich, mit dem erfindungsgemäßen System Reihenuntersuchungen durchzuführen. Der erfindungsgemäße Test kann auch sehr kostengünstig konzipiert werden, indem z.B. bekannte Detektionsmittel, z.B. chromogene, fluorogene, radioaktive, enzymatische oder luminogene Mittel, eingesetzt werden, womit eine aufwendige Analyse der Testergebnisse vermieden werden kann.

Das erfindungsgemäße Verfahren kann in herkömmlicher Weise in homogener Lösung durchgeführt werden. Bevorzugterweise wird aber das Verfahren derart durchgeführt, dass das Protein vorzugsweise vor der Bindung zum Liganden an einen festen Träger immobilisiert wird. Bevorzugterweise wird dabei das Protein mittels eines spezifischen Antikörpers an den festen Träger immobilisiert.

Bei anderen Ausführungsvarianten kann auch zunächst der Komplex aus Protein und Ligand gebildet werden und dieser Komplex spezifisch detektiert werden (etwa durch Immobilisierung oder Fällung des Komplexes bzw. spezifische Adsorption des Komplexes an einen festen Träger bzw. einem Detektionsagens).

Bei einer bevorzugten Verfahrensvariante ist der Ligand mit einem zur Detektion geeigneten Label vorgesehen. Dabei sind alle Labelarten geeignet, bevorzugterweise ist der Label ausgewählt aus der Gruppe Chromogen, Fluorogen, Radionuklid, Enzym, Lanthanid und Luminogen.

Zur Bestimmung des Ausmaßes an gebundenem Liganden können zwar spezifische Detektionsreagentien für den Komplex aus Ligand und Protein herangezogen werden. Bevorzugterweise wird das Ausmaß an gebundenem Liganden durch die Bestimmung des gebundenen Liganden selbst oder des gebundenen Proteins ermittelt. Es kann sich bei dieser Bestimmung um einen direkten oder indirekten Nachweis handeln.

Die für die jeweilige Bestimmung und Detektion notwendigen optimalen Bedingungen können selbstverständlich von jedem Fachmann auf diesem Gebiet leicht festgelegt werden. Beispielsweise kann ein bevorzugtes Bestimmungsverfahren so durchgeführt werden, dass das Protein an einen festen Träger gebunden wird, wobei dieser Träger nur eine limitierte Zahl an Bindungsstellen für das Protein aufweist und die Bindungsstellen gegenüber der Menge an Protein in der Probe limitiert sind und danach die Bindung des Liganden durchgeführt wird. Diese Ausführungsform hat den Vorteil, dass durch die prinzipiell limitierte Zahl an Bindungsstellen für das Protein eine gute Standardisierung ermöglicht wird. Als Standards werden beispielsweise Plasmen oder Präparate verwendet, die entweder nur Wildtyp-Protein, nur das Protein mit der Mutation oder eine definierte Mischung davon enthalten. Als Mittel zum Vergleich der Bindung der Liganden an das Protein in einer Probe mit der Bindung der Liganden an das Protein in einem Standard kann erfindungsgemäß auch ein numerischer Wert herangezogen werden bzw. eine graphische Darstellung des Verhältnisses.

Das erfindungsgemäße Verfahren kann prinzipiell für die Bestimmung jeglicher Mutanten bzw. Mutationen herangezogen werden. Bevorzugterweise wird es aber im Bereich der Humandiagnostik eingesetzt, und hier vor allem auf dem Gebiet der Blut- bzw. Serumdiagnostik.

Gemäß einem weiteren Aspekt betrifft die vorliegende Erfindung ein Set zur Durchführung des Verfahrens nach Anspruch 1, welches zumindest die folgenden Komponenten umfasst:

- einen Liganden, der entweder an das Wildtyp-Protein oder an das Protein mit einer Mutation bindet,

- einen festen Träger, welcher Bindungsstellen für das Protein aufweist,

- ein Mittel zur Detektion der Bindung des Liganden an das Protein, und

- ein Mittel zum Vergleich der Bindung des Liganden an das Protein in einer Probe mit der Bindung des Liganden an das Protein in einem Standard.

Bevorzugterweise wird im Set bereits ein markierter Ligand zur Verfügung gestellt, vorzugsweise ein biotinylierter Ligand. Vorteilhafterweise umfasst das Set auch noch zusätzlich einen Proteinstandard, vorzugsweise ein Wildtyp-Protein.

Gemäß einer bevorzugten Ausführungsform des erfindungsgemäßen Sets umfasst der feste Träger einen Antikörper, der sowohl das Wildtyp-Protein als auch das Protein mit einer Mutation in gleicher Weise, beispielsweise mit gleicher Bindungskonstante, bindet.

Die Erfindung wird durch die nachfolgenden Beispiele, auf die sie jedoch nicht eingeschränkt sein soll, näher erläutert.

**Beispiele:**

**Beispiel 1 :** Faktor V/Faktor V-Leiden-Bestimmung nicht Teil der beanspruchten Erfindung

**a) Testprinzip**: Auf einer mit einem anti-Faktor V-Antikörper beschichteten Mikrotestplatte werden Faktor V- bzw. Faktor V-Leiden-haltige Plasmen inkubiert. Dabei muss die Mikrotestplatte einerseits und die Plasmaverdünnung andererseits so eingestellt sein, dass die natürlichen intra- und interindividuellen Schwankungen der Faktor V-Konzentration so ausgeglichen sind, dass alle Plasmen unabhängig von diesen Störgrößen im Bindungsplateau reagieren. Erst so ist garantiert, dass zwischen normalem und mutiertem Faktor V unterschieden werden kann.

Der Test kann beispielsweise als Kombinationsassay durchgeführt werden. In einer ersten Reaktion wird der initial gebundene Faktor V zur Kontrolle mit einem mit alkalischer Phosphatase markierten anti-Faktor V-Antikörper als Faktor V-Antigen bestimmt. Der Nachweis der alkalischen Phosphatase erfolgt spektrophotometrisch mittels p-Nitrophenol als Farbstoff. Die Bestimmung einer ggfs. vorhandenen Mutation im Faktor V an der Aminosäureposition 506 (=Faktor V-Leiden-Mutation), also in einem für die Reaktion mit Protein C bzw. aktiviertem Protein C funktionell bedeutsamen Teil des Moleküls, erfolgt in einer zweiten Reaktion unter Verwendung eines von Protein C abgeleiteten, biotinylierten Peptides, das nur an den Faktor V, nicht aber an den Faktor V-Leiden bindet. Das biotinylierte .... Peptid wird dann mit einem mit Peroxidase markierten Streptavidin spektrophotometrisch bestimmt. Plasmen homozygoter Faktor V Leiden-Genträger sind im Antigentest positiv und im funktionellen Teil negativ. Plasmen Heterozygoter werden respektive wiederum im Antigenteil positiv und funktionell schwach nachweisbar.

**b) Auffinden der Liganden:** Im ersten Vorversuch wurden die Bindungsstellen des Faktor V für das den Faktor inaktivierende Enzym Protein C gesucht. Dabei wurde ein Peptid-Scan über die gesamte Aminosäuresequenz des Faktor V durchgeführt. Hierfür wurden 554 Peptide à 13 Aminosäuren (um jeweils 4 Aminosäuren versetzt) mit bekannten Verfahren der Peptidchemie hergestellt und auf einem Papierträger kovalent gebunden. Dieser Träger wurde nun mit einem biotinylierten Protein C, dessen funktionelle Aktivität zuvor mittels Bestimmung mit chromogenem Substrat nach Aktivierung mit PROTAC (Immunochrom Protein C®- Test der Firma IMMUNO AG, Wien) bestätigt wurde, inkubiert. Das Biotin wurde gemäß der Methode nach Kießig S.T., 1992 («Immunchemische Charakterisierung multireaktiver monoklonaler Antikörper", Habilitationsschrift, Humbold-Universität) mit einem mit alkalischer Phosphatase markierten Streptavidin und 5-Bromo-4-Chloro-3-Indolyl-Phosphat (BCIP) als Farbstoff spektrophotometrisch nachgewiesen. Dabei wurden die folgenden Sequenzen als PC-bindend gefunden (AA von Signalsequenz 1-28 gezählt):

AA 1-44
AA 62-81
AA 93-100
AA 118-129
AA 185-196
AA 210-212
AA 230-233
AA 305-308
AA 330-353 (=Schnittstelle I: ⇒AA R306+28= R334)

AA 365-401
AA 417-453
AA 457-484
AA 501-513
AA 517-545 (=Schnittstelle II: ⇒ AA R506+28= R534: R506 ⇒ Q506 = Faktor VLeiden)
AA 553-565
AA 621-649
AA 657-685
AA 697-713 (=Schnittstelle III: ⇒ AA R707+28= R735)
AA 725-745
AA 772-780
AA 794-797
AA 801-825
AA 841-853
AA 857-905
AA 913-923
AA 934-957
AA 968-1028
AA 1056-1059
AA 1062-1067
AA 1137-1149
AA 1217-1229
AA 1251-1264
AA 1492-1496
AA 1514-1517
AA 1545-1552
AA 1569-1575
AA 1598-1628
AA 1657-1677
AA 1741-1762
AA 1768-1772
AA 1785-1801
AA 1805-1821
AA 1825-1837
AA 1861-1876
AA 1914-1917
AA 1925-1934
AA 1977-1984
AA 1997-2021
AA 2027-2061
AA 2065-2067
AA 2086-2152
AA 2166-2168
AA 2186-2213

Die entscheidenen Schnittstellen R306=R334, R506=R534 und R707=R735 wurden von Protein C ebenfalls erkannt.

Da die Mutation des Faktor V-Leiden (R506Q) bekannt ist, wurde auf einer Mutationsanalyse dieser Region (KSRSLDRRGIQRAAD) die Bindung von Protein C analog dem bereits beschriebenen Verfahren überprüft. Dazu wurde jeweils eine AA gegen alle anderen natürlich vorkommenden ausgetauscht. Von besonderem Interesse war die Position R506 mit dem Austausch Q506. Es konnte festgestellt werden, daß der Austausch durch E bzw. D (Asparaginsäure bzw. Glutaminsäure) an keiner (außer der natürlichen) Position des gewählten Faktor V-Peptides toleriert wird, was bedeutet, das das Protein C nicht mehr oder nur sehr schwach bindet. Wird an der Position R506 mutiert, so fällt auf, daß der Ersatz durch Q zum Bindungsverlust führt. Austausche gegen A, E, H, P führen zum gleichen Ergebnis. Durch Austausche gegen C, D, F, G, I, M, N, S, V wird Protein C kaum noch gebunden. Lediglich Austausche gegen T, W, Y verändern die Bindungseigenschaften kaum. Damit konnte auf Peptidebene für Faktor V nachgewiesen werden, daß die Faktor V-Leiden Mutation dazu führt, daß Protein C nicht mehr gebunden wird und somit Protein C Faktor V-Leiden nicht auf dem üblichen schnellen Weg inaktivieren kann.

Die Bindungstellen des Faktor V an Regionen des Protein C wurden ebenfalls untersucht. Zu diesem Zweck wurde wieder ein Peptid-Scan über das Protein C durchgeführt. Der Nachweis der Bindung erfolgte entweder mit einem biotinylierten Faktor V oder Inkubation der Peptid-Scanmembran mit Vollplasma bzw. Faktor V Mangelplasma (immunadsorbiert) indirekt mit einem markierten Kaninchen-anti-human-Faktor V-Antikörper. Dabei konnten folgende Sequenzen als stark bindend für Faktor V gefunden werden (AA von Signalsequenz 1-32 gezählt):

AA 265-277 (LEGYDLRRWEKWE)
AA 409-422 (HNYGVYTKVSRYL

Folgende Sequenzen wurden als schwach bindend für Faktor V identifiziert (AA von Signalsequenz 1-32 gezählt):

AA 217-229 (MTRRGDSPWQVVL)
AA 339-358 (TGWGYHSSREKEAKRNRTF)

Negative Kontrollen wurden nicht gebunden.
Im weiteren wurde der Faktor V durch das Peptid, das die Faktor V-Leiden-Mutationstelle enthält, ersetzt. Damit sollte die Bindungsregion, d.h. das potentielle aktive Zentrum, zu definieren sein. Ein entsprechend biotinyliertes Faktor V-Peptid (KSRSDRRGI-QRAAD-Bio) bzw. Kontrollpeptid (KSRSDRRGI-GRAAD-Bio) mit einer Mutation außerhalb der Position R506 mit einer starken Bindung zum Protein C, wurde für den Nachweis eingesetzt. Der Bindungsnachweis erfolgte wie oben beschrieben mit Streptavidin-alkalischer Phosphatase.
Dabei konnten folgende Sequenzen als stark bindend für Faktor V gefunden werden (AA von Signalsequenz 1-32 gezählt) :

AA 4-16 (LTSLLLFVATWGI, Signalsequenz)
AA 73-85 (FQNVDDTLAFWSK)
AA 166-178 (YKLGDDLLQCHP)
AA 279-304 (LDIKEVFVHPNYSKSTTDNDIALLH) mit der Konsensussequenz:

AA 288-293 HPNYSKS
AA 333-346 (GQETLVTGWGYHS)
AA 336-352 (TLVTGWGYHSSREKEA)
AA 427-439 (GLLHNYGVYTKVS)
AA 449-461 (IRDKEAPQKSWAP, C-Terminus)

Das biotinylierte Faktor V-Kontrollpeptid KSRSDRRGIGRAAD mit einer Mutation außerhalb der Position R506 und einer stärkeren Bindung des Protein C wurde ebenfalls mit den Protein C-Sequenzen zur Reaktion gebracht.
Dabei konnten folgende Sequenzen zusätzlich als stark bindend für Faktor V gefunden werden (AA von Signalsequenz 1-32 gezählt):
AA 265-277 (LEGYDLRRWEKWE)
Folgende Sequenzen wurden als schwach bindend für Faktor V identifiziert (AA von Signalsequenz 1-32 gezählt):
AA 336-352 (TLVTGWGYHSSREKEA)
Das geschilderte Verfahren kann in analoger Weise auch für alle anderen Mutationen angewendet werden, um an geeignete Liganden zu gelangen.

**c) Beschichtung der Mikrotestplatten:**

Mikrotestplatten (MTP; Greiner) werden mit 2 µg/ml eines anti-Faktor V-Antikörpers in 0,1 mol/l Carbonatpuffer, pH 9,6, für 16 h bei Raumtemperatur beschichtet. Nachfolgend wird die MTP entleert und mit einem Nachbeschichtungspuffer (0,15 mol/l NaCl, 0,1 % BSA, 0,1 % Saccharose) inkubiert. So hergestellte Mikrotestplatten sind nach dem Trocknen eingeschweißt mit einem Trockenbeutel bis zu 24 Monaten haltbar.
**d) Probenvorbereitung:** Zitratplasma, Kalibratoren und Kontrollen werden 1 : 4 mit PBS der Zusammensetzung 0,1 mol/l Phosphatpuffer, pH 7,2, 0,15 mol/l Nacl, 0,1% Tween 20 (von Serva) verdunnt.
**e) Probeninkubation:** Die verdünnten Zitratplasmen, die Kontrolle Normal bzw. die Kontrolle Faktor V-Leiden (je 100 µl/Well, jeweils in Doppelbestimmungen) werden auf der vorbereiteten Mikrotestplatte für 90 min bei Raumtemperatur inkubiert. Danach werden die Mikrotestplatten 3 mal à 5 min mit 200 µl/Well PBS, 0,1 % Tween 20 gewaschen.
**f) 1. Konjugatinkubation:** Diese Reaktion erfolgt mit zwei Konjugaten gleichzeitig. Dafür wird ein AP-markierter anti-Faktor V-Antikörper (1 µg/ml) mit dem biotinylierten Protein C-Peptid HPNYSKS (0,5 µg/ml) zusammen in PBS, 0,1 % Tween 20 gemischt und mit jeweils 100 µl/Well für wiederum 90 min auf den Mikrotestplatten inkubiert. Abschließend wird wiederum gewaschen.
**g) 2. Konjugatinkubation:** Das biotinylierte Peptid läßt sich mit einem POD-markierten Streptavidin nachweisen. Dazu wird dieses nach den Angaben des Herstellers (SIGMA) im PBS, 0,1 % Tween 20 verdünnt und mit 100 µl/Well auf der Mikrotestplatte für 15 min bei Raumtemperatur inkubiert.

**h) 1. Substratreaktion:** Der Nachweis des mit alakilischer Phosphatase markierten anti-Faktor V-Antikörpers ist mittels p-Nitrophenol und üblichen Rezepturen möglich. Diese Reaktion wird nicht gestoppt, sondern nach exakt 30 min am Mikrotestplatten-Photometer bei 405 nm vermessen. Der Meßwert ist proportional der Faktor V-Konzentration, unabhängig von dem Vorhandensein einer eventuellen Mutation.

**i) 2. Substratreaktion:** Nach einem weiterem Waschschritt folgt der Nachweis des gebundenen mit Peroxidase markierten Streptavidin, das wiederum das gebundene Protein C-Peptid repräsentiert. Die Substratreaktion erfolgt unter üblichen Bedingungen mit Tetramethylbenzidin(TMB) und $H_2O_2$ mit einem Reaktionsvolumen von 100 µl/Well. Die Reaktion wird nach 30 min mit 100 µl/Well mit einer, 1 mol/l $H_2SO_4$ gestoppt und am Mikrotestplatten-Photometer bei 450 nm vermessen. Der Meßwert ist proportional der Faktor V-Mutation.

**j) Auswertung**: Bei nicht vorhandener Mutation sind sowohl die Reaktion aus dem Antigen- als auch aus dem funktionellen Teil positiv. Plasmen homozygoter Faktor V-Leiden-Genträger sind im Antigentest positiv und im funktionellen Teil negativ. Plasmen Heterozygoter werden respektive wiederum im Antigenteil positiv und funktionell schwach nachweisbar. Die Bewertung wird wie folgt durchgeführt:

Bestimmung der OD405nm = ApoB-Antigen
Bestimmung der OD450nm = ApoB-Mutation
Ermittlung des Quotienten des KontrolleNormal aus

$$Q_{KontrolleNormal} = \frac{OD_{450nmMutation}}{OD_{405nmAntigen}} = 100\%$$

Ermittlung des Quotienten der Kontrolle ApoBMut aus

$$Q_{KontrolleApoBMut} = \frac{OD_{450nmMutation}}{OD_{450nmAntigen}} = 0\%$$

Ermittlung des Quotienten für die Analytplasmen aus

$$Q_{Plasma} = \frac{OD_{450nmMutation}}{OD_{405nmAntigen}} = P\%$$

Vergleich der Quotienten der Einzelplasmen mit denen der Kontrollplasmen:

1. $Q_{Plasma} \geq Q_{KontrolleNormal}$ - 20% = keine_Mutation

2. $Q_{KontrolleNormal}$ - 20% $\geq$ $Q_{Plasma}$ $\geq$ $Q_{KontrolleApoBMut}$ + 20% = Mutation_hetero-

zygot

3. $Q_{Plasma}$ $\leq$ $Q_{KontrolleApoBMut}$ + 20% = Mutation_holmozygot

Damit ist eine Zuordnung zu den drei möglichen Zuständen homozygot normal, homozygot Faktor V-Leiden, heterozygot möglich und als Information an den anfordernden Arzt weiterzugeben.

Der Test kann auch als einfacher Assay ohne gleichzeitige Antigen-Bestimmung durchgeführt werden. Hierfür wird der Antikörper in einer Menge von etwa 0,1 µg/ml, also in limitierender Menge, zu Verfügung gestellt bzw. die Probe weniger stark verdünnt. Die Inkubation wird wie bereits beschrieben durchgeführt, ebenso die 1.Konjugatreaktion, allerdings in Abwesenheit entsprechender Antikörper und auch die 2.Konjugatreaktion. Die Menge an gebundenem Peptid wird dann nach Durchführung der 2.Substratreaktion indirekt bestimmt.

**Beispiel 2:** Apolipoprotein (a) - Bestimmung nicht Teil der beanspruchten Erfindung

**a) Testprinzip:** Auf einer mit einem anti-ApoB-100-Antikörper beschichteten Mikrotestplatte werden ApoB-100-haltige Plasmen inkubiert. Dabei muß die Mikrotestplatte einerseits und die Plasmaverdünnung andererseits so eingestellt sein, daß die natürlichen intra- und interindividuellen Schwankungen der ApoB-100-Konzentration so ausgeglichen sind, daß alle Plasmen unabhängig von diesen Störgrößen im Bindungsplateau reagieren. Mit einem biotinylierten Peptid, das sich von Apolipoprotein(a) ableitet, wird das immobilisierte ApoB-100 inkubiert. Das biotinylierte Peptid wiederum läßt sich mit einem POD-markierten Streptavidin nachweisen.

Bei nicht vorhandenen Mutationen ist die Reaktion positiv. Vorhandene Mutationen zeichnen sich durch abgeschwächte bzw. keine Reaktion aus.

**b) Auffinden des Liganden:** Im ersten Vorversuch wurde ein Peptid gesucht das die bekannte LDL-Rezeptorbindungsstelle mit der anschließenden Heparinbindungstelle des ApoB repräsentiert. Mittels eines Peptid-Scans wurde die Aminosäuresequenz DALQYKLEGTTRLTRKRGLKLATALSLSNKFGSHNST identifiziert. Eine vermutete Mutation in diesem Bereich führt möglicherweise zum literaturbekannten funktionellen Defekt.

Im zweiten Vorversuch wurde ein Peptid in der Apolipoprotein(a)-Sequenz gesucht, welches an o.g. Sequenz bindet. Mit Peptid-Scan wurde die Aminosäuresequenz Apo(a) K9 YTTDPCVRWEY identifiziert. Wobei besonders XTTXPXXRXXX (X = alle anderen bekannten Aminosäuren) wichtig sind. Der Diagnose liegt die Annahme zugrunde, daß das bindende Apo(a)-Peptid Strukturhomologie zum ApoB/E-Rezeptor besitzt und ein Verlust der Bindungsfähigkeit dieses Peptids auch den Verlust oder eine Verminderung der Affinität zu Rezeptor zur Folge hat (Vega, G.L., Grundy, S.M.: In vivo evidence for reduced binding of low density lipoproteins to receptors as a cause of primary moderate hypercholesterolemia. J. Clin. Invest. 78: 1410-1414, 1986).

**Testdurchführung**

**c) Beschichtung:** Mikrotestplatten (Greiner) werden mit 2 μg/ml eines anti-ApoB-Antikörpers im 0,1 mol/l Carbonatpuffer pH 9,6 für 16 Stunden bei Raumtemperatur beschichtet. Nachfolgend wird die Mikrotiterplatte entleert und mit einem Nachbeschichtungspuffer (0,15 mol/l NaCl, 0,1% Casein, 0,1% Dextran) inkubiert. So hergestellte Mikrotestplatten sind nach dem Trocknen eingeschweißt mit einem Trockenbeutel bis zu 24 Monaten haltbar.

**d) Probenvorbereitung:** Zitratplasma, Kalibratoren und Kontrollen werden 1:4 mit PBS (siehe Beispiel 1), 0,1% Tween 20 (SERVA) verdünnt.

**e) Probeninkubation:** Die verdünnten Zitratplasmen, die Kontrolle-ApoBNormal bzw. die Kontrolle-ApoBMut (je 100 μl/Well, jeweils in Doppelbestimmungen) werden auf der vorbereiteten Mikrotestplatte für 90 min bei Raumtemperatur inkubiert. Danach werden die Mikrotestplatten 3 mal à 5 min mit 200 μl/Well PBS, 0,1 % Tween 20 gewaschen.

**f) 1. Konjugatinkubation:** Diese Reaktion erfolgt mit zwei Konjugaten gleichzeitig. Dafür wird ein mit alkalischer Phosphatase (AP) markierter anti-ApoB-Antikörper (1μg/ml) mit dem biotinylierten Apo(a)-Peptid z. B. YTTDPCVRWEY (0,5 μg/ml) zusammen in PBS, 0,1% Tween 20 gemischt und mit jeweils 100 μl/Well für wiederum 90 min auf den Mikrotestplatten inkubiert. Abschließend wird wiederum gewaschen.

**g) 2. Konjugatinkubation:** Das biotinylierte Peptid läßt sich mit einem POD-markierten Streptavidin nachweisen. Dazu wird dieses nach den Angaben des Herstellers (SIGMA) im PBS, 0,1% Tween 20 verdünnt und mit 100μl/Well auf der Mikrotestplatte für 30 min bei Raumtemperatur inkubiert.

**h) 1. Substratreaktion:** Der Nachweis des AP-markierten anti-ApoB-Antikörpers ist mittels p-Nitrophenol (pNP) und üblichen Rezepturen möglich. Diese Reaktion wird nicht gestoppt, sondern nach exakt 30 min am Mikrotestplatten-Photometer bei 405nm vermessen. Der Meßwert ist proportional der ApoB-Konzentration, unabhängig von dem Vorhandensein einer eventuellen Mutation.

**i) 2. Substratreaktion:** Nach einem weiterem Waschschritt folgt der Nachweis des gebundenen mit Peroxidase (POD) markierten Streptavidin, das wiederum das gebundene Apo (a) -Peptid repräsentiert. Die Substratreaktion erfolgt unter üblichen Bedingungen mit TMB und $H_2O_2$ mit einem Reaktionsvolumen von 100μl/Well. Die Reaktion wird nach 30 min mit 100μl/Well mit einer 1mol/l $H_2SO_4$ gestoppt und am Mikrotestplatten-Photometer bei 450nm vermessen. Der Meßwert ist proportional der ApoB-Mutation.

**j) Auswertung:** Bei nicht vorhandener Mutation sind sowohl die Reaktion aus dem Antigen- als auch aus dem funktionellen Teil positiv. Plasmen homozygoter ApoBMut-Genträger sind im Antigentest positiv und im funktionellen Teil negativ. Plasmen Heterozygoter werden respektive wiederum im Antigenteil positiv und funktionell schwach nachweisbar. Die Bewertung wird wie folgt durchgeführt:

Bestimmung der OD405nm = ApoB-Antigen
Bestimmung der OD450nm = ApoB-Mutation
Ermittlung des Quotienten des KontrolleNormal aus

$$Q_{KontrolleNormal} = \frac{OD_{450nmMutation}}{OD_{405nmAntigen}} = 100\%$$

Ermittlung des Quotienten der Kontrolle ApoB-Mut aus

$$Q_{KontrolleApoBMut} = \frac{OD_{450nmMutation}}{OD_{405nmAntigen}} = 0\%$$

Ermittlung des Quotienten für die Analytplasmen aus

$$Q_{Plasma} = \frac{OD_{450nmMutation}}{OD_{405nmAntigen}} = P\%$$

Vergleich der Quotienten der Einzelplasmen mit denen der Kontrollplasmen:

1. $Q_{Plasma} \geq Q_{KontrolleNormal}$ - 20% = keine_Mutation
2. $Q_{KontrolleNormal}$ - 20% ≥ $Q_{Plasma}$ ≥ $Q_{KontrolleApoBMut}$ + 20% = Mutation_heterozygot
3. $Q_{Plasma} \leq Q_{KontrolleApoBMut}$ + 20% =

Mutation_homozygot

Damit ist eine Zuordnung zu den drei möglichen Zuständen homozygot normal, homozygot ApoBMut, heterozygot möglich und als Information an den anfordernden Arzt weiterzugeben.

**Patentansprüche**

1. Verfahren zur Bestimmung eines Proteins mit einer Mutation in einer Probe, die gegebenenfalls den Wildtyp des Proteins enthält, durch Zusetzen eines Liganden zur Probe, welcher Ligand entweder an das Protein mit der Mutation bindet oder an den Wildtyp, Ermitteln des Ausmaßes an gebundenem Liganden und Qualifizieren bzw. Quantifizieren der Mutation, **dadurch gekennzeichnet, dass** das Protein der Blutgerinnungsfaktor VII ist.

2. Verfahren nach Anspruch 1, **dadurch gekennzeichnet, dass** als Ligand ein Peptid zugesetzt wird.

3. Verfahren nach Anspruch 1 oder 2, **dadurch gekennzeichnet, dass** ein Ligand zugesetzt wird, der von einem physiologischen Bindungspartner zum Wildtyp abgeleitet ist.

4. Verfahren nach einem der Ansprüche 1 bis 3, **dadurch gekennzeichnet, dass** ein Ligand zugegeben wird, der ein Peptid mit wenigstens drei Aminosäuren ist.

5. Verfahren nach einem der Ansprüche 1 bis 4, **dadurch gekennzeichnet, dass** ein Ligand zugegeben wird, der wenigstens eine unphysiologische Aminosäure umfasst.

6. Verfahren nach einem der Ansprüche 1 bis 5, **dadurch gekennzeichnet, dass** als Ligand ein Antikörper zugegeben wird, der an die Stelle der Mutation bindet.

7. Verfahren nach einem der Ansprüche 1 bis 6, **dadurch gekennzeichnet, dass** ein Ligand, der mit einem zur Detektion geeigneten Label versehen ist, zugegeben wird.

8. Verfahren nach einem der Ansprüche 1 bis 7, **dadurch gekennzeichnet, dass** das Label ausgewählt ist aus der Gruppe Chromogen, Fluorogen, Radionuklid, Enzym, Lanthanid und Luminogen.

9. Verfahren nach einem der Ansprüche 1 bis 8, **dadurch gekennzeichnet, dass** das Protein an einem festen Träger immobilisiert ist.

10. Verfahren nach einem der Ansprüche 1 bis 9, **dadurch gekennzeichnet, dass** das Protein mittels eines spezifischen Antikörpers an den festen Träger immobilisiert wird, welcher Antikörper nicht an die Stelle der Mutation bindet.

11. Verfahren nach einem der Ansprüche 1 bis 10, **dadurch gekennzeichnet, dass** das Protein eine funktionelle Mutation aufweist.

12. Verfahren nach einem der Ansprüche 1 bis 11, **dadurch gekennzeichnet, dass** das Ausmaß an gebundenem Liganden durch Detektion des gebundenen Liganden und/oder gebundenen Proteins ermittelt wird.

13. Set zur Durchführung des Verfahrens nach Anspruch 1, umfassend die Komponenten:

    - einen Liganden, der entweder an das Wildtyp-Protein oder an das Protein mit einer Mutation bindet,
    - einen festen Träger, welcher Bindungsstellen für das Protein aufweist,
    - ein Mittel zur Detektion der Bindung des Liganden an das Protein, und
    - ein Mittel zum Vergleich der Bindung des Liganden an das Protein in einer Probe mit der Bindung des Liganden an das Protein in einem Standard.

14. Set nach Anspruch 13, **dadurch gekennzeichnet, dass** der Ligand mit einem Label versehen ist.

15. Set nach Anspruch 13 oder 14, **dadurch gekennzeichnet, dass** es zusätzlich einen Standard umfasst.

16. Set nach einem der Ansprüche 13 bis 15, **dadurch gekennzeichnet, dass** der feste Träger einen Antikörper umfasst, der sowohl den Wildtyp als auch das Protein mit einer Mutation bindet.

**Claims**

1. A method of determining a protein having a mutation in a sample, which sample possibly includes the wild type of the protein, by adding a ligand to the sample, which ligand binds either to the protein having said mutation or to the wild type, determining the amount of ligand bound, and qualifying and quantitating, respectively, said mutation, **characterised in that** said protein is blood coagulation factor VII.

2. A method according to claim 1, **characterised in that** a peptide is added as said ligand.

**3.** A method according to claim 1 or 2, **characterised in that** a ligand derived from a physiological binding partner to said wild type is added.

**4.** A method according to any one of claims 1 to 3, **characterised in that** a ligand which is a peptide comprising at least three amino acids is added.

**5.** A method according to any one of claims 1 to 4, **characterised in that** a ligand comprising at least one unphysiological amino acid is added.

**6.** A method according to any one of claims 1 to 5, **characterised in that** an antibody which binds to the site of the mutation is added as said ligand.

**7.** A method according to any one of claims 1 to 6, **characterised in that** a ligand which has been provided with a label suitable for detection is added.

**8.** A method according to any one of claims 1 to 7, **characterised in that** said label is selected from the group chromogen, fluorogen, radionuclide, enzyme, lanthanoid and luminogen.

**9.** A method according to any one of claims 1 to 8, **characterised in that** the protein is immobilised on a solid carrier.

**10.** A method according to any one of claims 1 to 9, **characterised in that** the protein is immobilised on the solid carrier by means of a specific antibody, which antibody does not bind to the site of the mutation.

**11.** A method according to any one of claims 1 to 10, **characterised in that** the protein has a functional mutation.

**12.** A method according to any one of claims 1 to 11, **characterised in that** the amount of bound ligand is determined by detecting the bound ligand and/or the bound protein.

**13.** A kit for carrying out the method according to claim 1, comprising the components

- a ligand either binding to the wild type protein or to the protein having a mutation,
- a solid carrier having binding sites for the protein,
- means for detecting the binding of the ligand to the protein, and
- means for comparing the binding of the ligand to the protein in a sample with the binding of the ligand to the protein in a standard.

**14.** A kit according to claim 13, **characterised in that** the ligand is provided with a label.

**15.** A kit according to claim 13 or 14, **characterised in that** it additionally comprises a standard.

**16.** A kit according to any one of claims 13 to 15, **characterised in that** the solid carrier comprises an antibody binding to both the wild type and to the protein having a mutation.

**Revendications**

**1.** Procédé de détermination d'une protéine avec une mutation dans un échantillon, qui contient le cas échéant le type sauvage de la protéine, par ajout à l'échantillon d'un ligand qui se lie soit à la protéine avec la mutation, soit au type sauvage, détermination de la quantité de ligand lié et qualification ou quantification de la mutation, **caractérisé en ce que** la protéine est le facteur de coagulation sanguine VII.

**2.** Procédé selon la revendication 1, **caractérisé en ce que** l'on ajoute comme ligand un peptide.

**3.** Procédé selon la revendication 1 ou la revendication 2, **caractérisé en ce que** l'on ajoute un ligand qui est dérivé d'un partenaire de liaison physiologique du type sauvage.

**4.** Procédé selon l'une des revendications 1 à 3, **caractérisé en ce que** l'on ajoute un ligand qui est un peptide avec au moins trois acides aminés.

**5.** Procédé selon l'une des revendications 1 à 4, **caractérisé en ce que** l'on ajoute un ligand qui comprend au moins un acide aminé non physiologique.

**6.** Procédé selon l'une des revendications 1 à 5, **caractérisé en ce que** l'on ajoute comme ligand un anticorps qui se lie au site de la mutation.

**7.** Procédé selon l'une des revendications 1 à 6, **caractérisé en ce que** l'on ajoute un ligand qui est doté d'un marqueur approprié pour la détection.

**8.** Procédé selon l'une des revendications 1 à 7, **caractérisé en ce que** le marqueur est choisi dans le groupe des chromogènes, fluorogènes, radionucléides, enzymes, lanthanides et luminogènes.

**9.** Procédé selon l'une des revendications 1 à 8, **caractérisé en ce que** la protéine est immobilisée sur un support solide.

**10.** Procédé selon l'une des revendications 1 à 9, **caractérisé en ce que** la protéine est immobilisée sur le support solide au moyen d'un anticorps spécifique qui ne se lie pas au site de la mutation.

11. Procédé selon l'une des revendications 1 à 10, **caractérisé en ce que** la protéine présente une mutation fonctionnelle.

12. Procédé selon l'une des revendications 1 à 11, **caractérisé en ce que** la quantité de ligand lié est déterminée par détection du ligand lié et/ou de la protéine liée.

13. Kit pour la réalisation du procédé selon la revendication 1, comprenant les composants suivants :

   - un ligand qui se lie soit à la protéine de type sauvage, soit à la protéine avec une mutation,
   - un support solide qui présente des sites de liaison pour la protéine,
   - un moyen pour détecter la liaison du ligand à la protéine, et
   - un moyen pour comparer la liaison du ligand à la protéine dans un échantillon à la liaison du ligand à la protéine dans un étalon.

14. Kit selon la revendication 13, **caractérisé en ce que** le ligand est doté d'un marqueur.

15. Kit selon la revendication 13 ou la revendication 14, **caractérisé en ce qu'**il comprend en plus un étalon.

16. Kit selon l'une des revendications 13 à 15, **caractérisé en ce que** le support solide comprend un anticorps qui se lie aussi bien au type sauvage qu'à la protéine avec une mutation.